(19) Europäisches Patentamt / European Patent Office / Office européen des brevets

(11) **EP 4 575 477 A1**

(12) **EUROPEAN PATENT APPLICATION**

(43) Date of publication:
**25.06.2025 Bulletin 2025/26**

(21) Application number: **23219911.7**

(22) Date of filing: **22.12.2023**

(51) International Patent Classification (IPC):
**G01N 24/08** (2006.01)    **G01R 33/31** (2006.01)
**G01R 33/44** (2006.01)    **G01N 33/00** (2006.01)
**G01N 33/44** (2006.01)    **H01L 31/048** (2014.01)
**G01R 33/30** (2006.01)    **G01R 33/46** (2006.01)

(52) Cooperative Patent Classification (CPC):
**G01R 33/448; G01N 24/08; G01N 33/0096;**
**G01N 33/442; G01R 33/31; H10F 19/80;**
G01N 24/085; G01N 2203/0092; G01R 33/307;
G01R 33/445; G01R 33/4625

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Fraunhofer-Gesellschaft zur Förderung der angewandten Forschung e.V. 80686 München (DE)**

(72) Inventor: **Mordvinkin, Anton 06120 Halle (DE)**

(74) Representative: **Friese Goeden Patentanwälte PartGmbB Widenmayerstraße 49 80538 München (DE)**

(54) **METHOD AND DEVICE FOR NON-DESTRUCTIVE ANALYSIS OF POLYMERIC SAMPLES USING MAGNETIC RESONANCE SENSORS**

(57) The invention relates to a method for analysing a polymer sample (2) with nuclear magnetic resonance (NMR), the method comprising the following steps:

providing a polymer sample (2);

tempering a polymer sample (2);

measuring a surface temperature of the polymer sample (2);

subjecting the polymer sample (2) to a magnetic field of a magnet (4);

applying a radiofrequency pulse sequence to the polymer sample (2);

obtaining a radiofrequency signal response from the polymer sample (2);

determining a microstructure-dependent parameter of the polymer sample (2) on the basis of the signal response.

Fig. 2

**Description**

The present invention is directed towards a method and a device for non-destructive analysis of a polymer sample. In particular, the present invention is directed towards a method and a system for non-destructive analysis of crosslinking, crystallinity and water content in polymer samples, in particular in photovoltaic (PV) modules and safety glasses.

Photovoltaic modules can be seen as polymer samples comprising a front glass substrate, a first polymeric encapsulant sheet (e.g. ethylene-vinyl acetate copolymer (EVA)), photovoltaic cells, and a second polymeric encapsulant sheet stacked and laminated together. On the backside of the polymeric encapsulant sheet there is either a polymer backsheet or a second glass substrate laminated to the second polymeric encapsulant sheet: In the first case the photovoltaic module is said to be a glass-backsheet module and in the second case the photovoltaic module is said to be a glass-glass module. The encapsulant sheets (which might also be referred to as polymer encapsulants or encapsulation films) might also be formed by other polymers, such as polyolefin elastomers, silicones, polyurethanes, ionomers, etc. Safety glasses consist of two glass substrates connected through lamination of one or several layers of polymeric encapsulants, placed in between the glass substrates.

The lifetime and reliability of photovoltaic modules as well as of safety glasses described above depends on the lamination quality and the associated degree of crosslinking of the encapsulation films during manufacturing. An enhanced lifetime contributes to reduction of energy costs and improvement of sustainability and economical attractiveness of photovoltaic modules and safety glasses.

A current method for testing the quality, and in particular the lamination quality, of photovoltaic modules is, for example, the Soxhlet extraction (or swelling experiments). Such method requires however the destruction of the photovoltaic module and is time consuming by requiring times of up to 24 hours. Also, the Soxhlet extraction is not well suited for glass-glass photovoltaic modules or for safety glasses due to complicated sample extraction.

Other destructive methods used in the quality, and in particular in the lamination control of polymeric samples, are differential scanning calorimetry and dynamic mechanical analysis.

In the prior art there are also some non-destructive methods, such as Raman spectroscopy, luminescence spectroscopy and thermomechanical indentation. Such methods show promising results, but also have several drawbacks: in the first place they can estimate the lamination quality at only one side of the photovoltaic panel (either the front or the back side), thus impairing a complete lamination control, which is important in particular due to the thermal gradient brought about by the laminator in the photovoltaic panel. Also, such methods were shown to work well for only one type of the encapsulation films, namely for ethylene-vinyl acetate copolymer films, but were not yet shown to work for other encapsulation films, such as polyolefin elastomers. Further, some of the methods (thermomechanical indentation) are intrinsically not adapted for glass-glass photovoltaic modules, which are gaining more and more importance in the market.

Some promising results have been shown by using nuclear magnetic resonance on polymer samples and on photovoltaic modules to estimate the crosslinking degree as well as the degradation state of the sample or of the module. Such method has been however applied only in a destructive manner for measurements of the lamination of ethylene vinyl acetate samples and is designed to merely decouple the lamination problems associated with the lamination process and material formulation.

Therefore, there is the need for providing a method for ensuring a fast and non-destructive lamination control in polymer samples, and in particular in photovoltaic modules, for ensuring high quality of produced polymer samples of photovoltaic modules and/or safety glasses.

The above defined need is solved by a method according to the first aspect of the present invention and by a device according to the second aspect of the present invention.

Advantageous embodiments of the present invention are object of the dependent claims.

According to a first aspect of the present invention a method for analysing a polymer sample is provided. The method comprises the following steps: providing a polymer sample; tempering the polymer sample; measuring a surface temperature of the polymer sample; subjecting the polymer sample to a magnetic field of a magnet; applying a radiofrequency pulse sequence to the polymer sample; obtaining a radiofrequency signal response from the polymer sample; determining a microstructure-dependent parameter of the polymer sample on the basis of the signal response and/or on the basis of a comparison with reference information.

According to the present invention, a polymer sample can be a monolayer polymer sample or a multilayer polymer sample. A multilayer polymer sample comprises at least two layers of polymeric material such as, for example, but not limited to ethylene vinyl acetate or polyolefin elastomer. Within the meaning of the present invention, a polymeric sample can also comprise glass and other inorganic components such as silicon-based solar cells.

A polymer sample can therefore also be a photovoltaic module comprising a first glass substrate on the frontside of the photovoltaic module, a first encapsulation film, such as ethylene vinyl acetate or polyolefin elastomer, a photovoltaic cell, a second encapsulation film and a polymeric backsheet or a second glass substrate on the backside of the photovoltaic module.

A polymer sample within the meaning of the present invention can also be safety glasses, comprising a polymeric film being sandwiched between two glass substrates.

Tempering the polymer sample means within the scope of the present invention, that the polymeric sample is heated up at a constant temperature prior or during to subjecting the polymer sample to the magnetic field of the magnet. The temperature range might vary between 20 ° and 200 °. The tempering of the polymer sample might be performed by means of a tempering device after the lamination of the polymer sample.

The method according to the first aspect of the present invention is therefore based on the nuclear magnetic resonance spectroscopy principle. Accordingly, the polymer sample is subjected to a magnetic field and a radiofrequency pulse sequence. The radiofrequency pulse sequence perturbates the nuclei of the polymer sample, which respond by emitting an electromagnetic signal. The signal (response) can be used to characterize the microstructure of the polymer sample. The radiofrequency pulses in the pulse sequence can have different frequencies ranging between 0.5 and 1000 kHz, depending on the magnet sensor system design.

The reference information represents a calibration of the microstructure-dependent parameter performed before the method is carried out.

In particular, using a suitable pulse sequence, the signal response can be obtained and sampled to determine the spin-lattice relaxation time ($T_1$), the spin-spin relaxation time ($T_2$, more exactly $T_{2eff}$ in the inhomogeneous external and polarization magnetic fields), the proton density of the polymer sample, or/and the dipole-dipole couplings. From one or more of the above cited parameters, the microstructure of the polymer sample can be determined.

According to an embodiment, the pulse sequence is a CPMG (Carr-Purcell-Meiboom-Gill) pulse sequence. Other pulse sequences can also be used in the method according to the invention for retrieving the $T_1$, multiple-quantum (or double quantum) nuclear magnetic resonance for dipole-dipole couplings.

The microstructure-dependent parameter determined on the basis of the signal response might in particular be related to the cross-linking degree within the polymer sample.

The temperature of the polymer sample, at least of a surface thereof, is measured prior to applying the pulse sequence, due to the dependency of the signal response on temperature. In particular, it should be noted that the signal response during a nuclear magnetic resonance spectroscopy measurement is temperature dependent due to the changes in the molecular mobility of the polymer sample, which turn into the averaging out specific information, primarily residual dipolar coupling, driving the signal decay. Therefore, knowledge of the polymer sample temperature is fundamental for obtaining a reliable result from the method according to the first aspect of the present invention. According to embodiments of the present invention, the temperature of the of the polymer sample might be monitored during the whole execution of the method for providing a more reliable correlation between the signal response and the measured temperature.

The radiofrequency pulse sequence applied to the polymer sample can be emitted from an emitter-receiver (radiofrequency coil), which is also configured for receiving the signal response.

The invention is therefore based on the main idea that nuclear magnetic resonance might be used for analysing the lamination and degradation state of the polymer sample. Such technique has shown, that the method exhibits a high sensitivity to the microstructure of the polymer sample, in particular as far crosslinking density or degree, crystallinity and water content are regarded.

Another major advantage recognized by the inventors is that, with the method according to the first aspect of the present invention, it is possible to analyse glass-glass photovoltaic modules as well as safety glasses due to the non-destructive nature of the nuclear magnetic resonance spectroscopy being applied to the polymer sample.

Furthermore, it was possible to show that the method according to the first aspect of the present invention allows different kind of photovoltaic modules with different encapsulation films such as, but not limited to, ethylene-vinyl acetate copolymer, polyolefin elastomer, silicones, polyurethane and ionomers.

Therefore, it is possible with the method according to the first aspect of the present invention to have an efficient non-destructive methodology for analysing the microstructure such as the crosslinking degree, crystallinity, and the water content in different polymer samples. This enables performing an in-situ lamination quality control and assessment of the degradation state.

The method according to the first aspect of the present invention can be applied both to inline lamination processes, where polymer samples are fed in a continuous manner to a device for carrying out the method, as well as to offline control processes, where a polymer sample is analysed at a location physically separated from the lamination line.

In the latter case, a thorough, high resolution three-dimensional mapping of the microstructure of the polymer sample across the sample surface and its depth might be obtained, so that a detailed non-destructive in-situ analysis of polymer lamination and aging state can be conducted. The aging state of the polymer sample defined by post-crystallization, chain-splitting, post-cross-linking, and water ingress, which precede macroscopic changes leading to backsheet cracking, delamination, yellowing and corrosion of the metallic contacts, can be easily assessed. Thus, it is now possible to better identify and analyse parameters having a dramatic influence over the lifetime of the photovoltaic module.

Summarizing, the method according to the first aspect of the present invention can be applied both to the lamination control during the manufacturing process of the polymer sample as well as during the whole lifetime of the polymer sample, during which the degradation state of the polymer sample is investigated.

According to an embodiment, the polymer sample is a multilayer sample with at least two layers, wherein the two layers are in particular an ethylene vinyl acetate layer and a polyolefin elastomer layer. Therefore, polymer samples having a multilayer structure can be analysed in a layer-by layer procedure, thus allowing the possibility of enacting quality controls over multilayer polymer samples without the need for a destructive method. The contrast between the different layers is brought about by different spin-spin relaxation times, which are characteristic of every material, and which are intrinsically defined by the intrasegmental arrangement of protons as well as segmental mobilities.

The multilayer polymer sample can also comprise an ethylene vinyl acetate layer flanked by or sandwiched between two polyolefin elastomer layers.

According to an embodiment, the polymer sample is photovoltaic module, in particular a glass-backsheet module or a glass-glass module, where all the polymer layers (encapsulants and backsheets) can be analyzed. According to an embodiment, the photovoltaic module also includes a photovoltaic cell having a paramagnetic centre, normally originating from the aluminium within the photovoltaic cell.

Also, the multilayer polymer sample can be a security glass comprising a front glass layer, a polymer layer and a second glass layer, wherein the polymer layer is arranged between the first glass layer and the second glass layer.

According to a further embodiment, the method comprises extracting the spin-lattice relaxation time ($T_1$) from the signal response, after the signal response has undergone an inversion recovery or a saturation recovery. To this purpose, it is particularly advantageous to use a CPMG pulse sequence to excite the polymer sample.

According to a further embodiment, the method comprises extracting the a proton density and/or a spin-spin relaxation time ($T_{2,eff}$) from the signal response.

According to a further embodiment, the method comprises extracting dipole-dipole couplings from the signal response. This can be achieved by means of a double-quantum experiment directly related to the cross-linking degree.

According to an embodiment, the method further comprises performing a signal fitting of the signal response, wherein the signal fitting is in particular based on a sum of exponentials, in particular of stretched exponentials.

Thus, the signal response can be fitted on a model that takes into consideration the presence of multiple phases within different segmental mobility of the polymer sample, such as crystallinity, network chains, network defects, water or other solvent.

In particular, such model of the signal fitting can be generalized by a sum of stretched exponential according to the following formula

$$S(t) \sim \sum_i A_i \exp\left(-\left(\frac{t}{T_{2,i}}\right)^{b_i}\right),$$

where $A_i$ is an amplitude of a component i, and bi is an exponent ranging between 0.5 and 2 ($b_i < 1$ corresponds to a distribution of $T_{2eff}$ times within the phase I, $b_i > 1$ means that the signal decay is mostly driven by dipole-dipole spin interactions).

Therefore, the fitting model enables the assessment of the relative content of each dynamic region and its mobility on the base of the spin-spin relaxation time. Thus, the result of the signal fitting model might be related to the microstructure-dependent parameter, such as, for example, the cross-linking degree of the polymer sample.

According to an embodiment, the method further comprises normalizing the signal response based on a microstructure-dependent parameter, to the maximum intensity at an initial time, which is a signal maximum related to the total spin density, and subsequently integrating the signal response over time to yield amplitude-weighted spin-spin relation times values, which is an amplitude-weighted $T_{2eff}$ average.

According to an embodiment, the method further comprises performing an echo-summation ($T_{2eff}$-weighted amplitude) of the signal response with an optional prior signal normalization by the signal maximum related to the total spin density. This approach can significantly improve the signal-to-noise ratio due to the addition of all points, thus enabling the analysis of low-intensity signals and reduction of measuring times. A reciprocal of the found $T_{2eff}$-weighted amplitude is related to the cross-linking density (and cross-linking degree, measured by the Soxhlet extraction) and is called in the following as the NMR cross-linking density.

According to an embodiment, the method further comprises inverting a $T_{2eff}$ weighted amplitude. The inventors have found out, that there is a strong correlation between the inverted valued of the $T_{2eff}$ weighted amplitude and the cross-linking degree. Other $T_{2eff}$ parameters can also be correlated with the cross-linking degree.

According to an embodiment, the method further comprises comparing the microstructure-dependent parameter with reference information. The reference information represents a calibration of the microstructure-dependent parameter performed before the method is carried out. For example, the cross-linking degree of a polymer sample having a particular composition can be determined for different cross-linking degrees (cross-linking time) by means of a Soxhlet extraction. Polymer samples having the same composition and being subjected to the same cross-linking time can be analysed by means of the method as of the first aspect of the present invention, after which a correlation between the cross-linking degree as determined by means of the Soxhlet extraction and the cross-linking degree as determined by the method as of the first aspect of the present invention is obtained. Therefore, all future polymer samples having the same composition can be analysed by means of the method as of the first aspect of the present invention and the result can be compared with the calibration obtained by correlating the results from the Soxhlet extraction and the method according to the first aspect of the present invention.

According to an embodiment, the non-invasive measurements can occur in stray magnetic fields of magnets with the magnet-field strength on the order of 0.1-1 T with defined gradients (5-100 T/m). A suitable sensor geometry and measurement parameters (90°-pulse length, acquisition time) ensure selection of an excited slice (volume) with a predefined thickness (on the order of 1-1000 $\mu$m) and area, depending on the r.f. coil size (on the order of 1x1-50x50 mm2), at the predefined depth in the sample (on the order of 0-30 mm). In such a way it is possible to identify changes of the microstructure-dependent parameter in a desired sample position with a desired resolution and sensitivity.

The magnetic field is a static magnetic field generated from a permanent magnet. Such magnetic field has an intrinsic dependence of its strength on the distance from the magnet surface (intrinsic gradient). Therefore, there is an inhomogeneous magnetic field with a defined gradient, fixed by the magnet design.

According to an embodiment, the gradient of the stray magnetic field is a one-dimensional, a two-dimensional or a three-dimensional gradient.

According to a second aspect of the present invention, a device for carrying out a method according to the first aspect of the present invention is provided. The device comprises a supporting structure for supporting the polymer sample; a temperature sensor for measuring a surface temperature of the polymer sample; a magnet generating a magnetic field at a location of the polymer sample; a radio-frequency coil for sending radio-frequency pulse sequences at the location of the polymer sample and for receiving a radiofrequency signal response of the polymer sample; a spectrometer for the pulse generation; and a computing device for experimental controlling and determining a microstructure-dependent parameter of the polymer sample on the basis of the signal response.

According to an embodiment, the device further comprises a heating device for heating the polymer sample, wherein it is preferably provided that the heating device is a halogen lamp or a hot-air ventilator. Other heating sources can be applied as well.

Therefore, the polymer sample can be heated-up or tempered for realizing constant high-temperatures, ranging between 20 and 200°C, being precisely determined (+/- 1°C), within the measured volume in the polymer sample, thus allowing a more precise response from the nuclear magnetic resonance spectroscopy. Consequently, the method according to the first aspect of the present invention might further comprise a step of tempering the polymer sample for obtaining constant high temperatures throughout the measured volume of the polymer sample. The polymer sample tempering is required for using in the offline mode or also optionally in the inline mode.

According to an embodiment, the device can also take advantage of high temperatures of the polymer samples coming out of the laminator in the inline configuration. To this end, the tempering step is not needed, but rather a precise and fast temperature control during the measurement at the variable temperature. The measurement will have to be fast to ensure a constant temperature at the moment of measurement with a permitted variability of 1-5 degrees. The permitted temperature variability can be adjusted depending on the analyzed polymer sample.

According to an embodiment, the device further comprises a further temperature sensor for measuring a temperature of the magnet. Thus, the temperature of the magnet can be taken into account during the nuclear magnetic resonance spectroscopy performed on the polymer sample.

According to an embodiment, the device further comprises a cooling device for cooling the magnet. Therefore, the magnet can be kept at a constant temperature by being cooled down, thus avoiding a temperature increase caused by the heat transferred from the polymer sample - or the surrounding environment - to it. Ideally, the magnet temperature is controlled to be less than 40 C for the whole process. Correspondingly, the method according to the first aspect of the present invention might further comprise a step of controlling the temperature of the magnet to be less than 40°C.

Also, the magnet can be placed within a shielding box for thermally decoupling it from the surrounding environment.

According to an embodiment, the device can further comprise a positioning device for moving the magnet along a direction being perpendicular to a surface of the polymer sample. Thus, the device can perform nuclear magnetic resonance spectroscopy scans at different depths of the polymer sample.

According to an embodiment, the device further comprises a supporting construction configured for allowing a relative movement between the polymer sample and the magnet. Therefore, the device can allow an inline analysis of a moving polymer sample exiting a laminator device. In this case, the microstructure-dependent parameter obtained from the computing device can be a spatial average over the sample area, depending on the moving velocity of the polymer sample as well as the size of the radio-frequency coil.

In the present invention, features related to the method according to the first aspect might be applied to the device according to the second aspect and features related to the device according to the second aspect might be applied to the method according to the first aspect.

In the following figures, the invention and the general idea behind it will be explained in a more detailed and nonlimiting manner.

Figure 1 shows a laminator with a measuring device according to the second aspect of the present invention.

Figure 2 shows a first embodiment of a device according to the second aspect of the present invention for the measurements in the inline and offline modes with the heating realized from the bottom of the polymer sample.

Figure 3 shows a second embodiment of a device according to the second aspect of the present invention for the measurements in the inline and offline mode with the heating realized from the top of the polymer sample.

Figure 4 shows schematically a diagram of a method according to an embodiment of the first aspect of the present invention.

Figure 5 shows a diagram of a signal response intensity over time for a polymer sample for different cross-linking times.

Figure 6 shows a diagram of a normalised NMR cross-linking degree, obtained by the method, for polymer samples having different cross-lining times.

Figure 7 shows a comparison of a normalised NMR cross-linking degree with a Soxhlet cross-linking degree determined using a Soxhlet extraction.

Figure 8 shows a nuclear magnetic resonance spectroscopy two-dimensional image of a specially laminated ethylene-vinyl acetate copolymer film as a polymer sample with introduced lamination defects.

Figure 9 shows a depth-profile of the normalized signal response intensity related to a cross-linking degree within a polymer sample having multiple layers, demonstrating the effect of a temperature gradient in the laminator over the polymer sample thickness.

Figure 10 shows a depth-profile of the normalized signal response intensity representing a cross-linking degree within another polymer sample having multiple layers of different polymer classes (EVA and POE).

Figure 11 shows a comparison of intensities of a normalized signal response intensity for a polyolefin elastomer polymer sample and for an ethylene-vinyl acetate polymer sample.

Figure 12 shows a diagram with the intensity of a signal response from a polymer sample with or without a photovoltaic cell in the sample proximity.

Figure 13 shows a normalised signal intensity for the polymer sample with or without the photovoltaic cell in the sample proximity as in Figure 12, wherein for the polymer sample with the photovoltaic cell the number of scans is increased by a factor 16.

Figure 14 shows a normalised signal intensity for a polymer sample with a photovoltaic cell for the case in which the number of scans is increased by a factor 16 or for the case in which the number of scans is not increased by a factor 16.

Figure 15 shows an NMR cross-linking degree, normalized at the maximal lamination time of 30 minutes, for a polymer sample with a photovoltaic cell against the cross-linking time for both the case in which the number of scans is increased by a factor 16 or for the case in which the number of scans is not increased by a factor 16.

Figure 16 shows an NMR cross-linking degree for a polymer sample with or without a photovoltaic cell against the cross-linking time.

Figure 17 shows a diagram plotting a normalised intensity of a signal response as measured in a polymer sample with a solar cell oriented with the sunny side towards the radiofrequency coil and oriented with the backside of the cell towards the radiofrequency coil.

Figure 18 represents schematically the NMR cross-linking degree as derived from the signal response against the cross-linking time for different temperatures.

Figure 19 represents schematically the NMR cross-linking degree as derived from the signal response against the sample temperature for different cross-linking times.

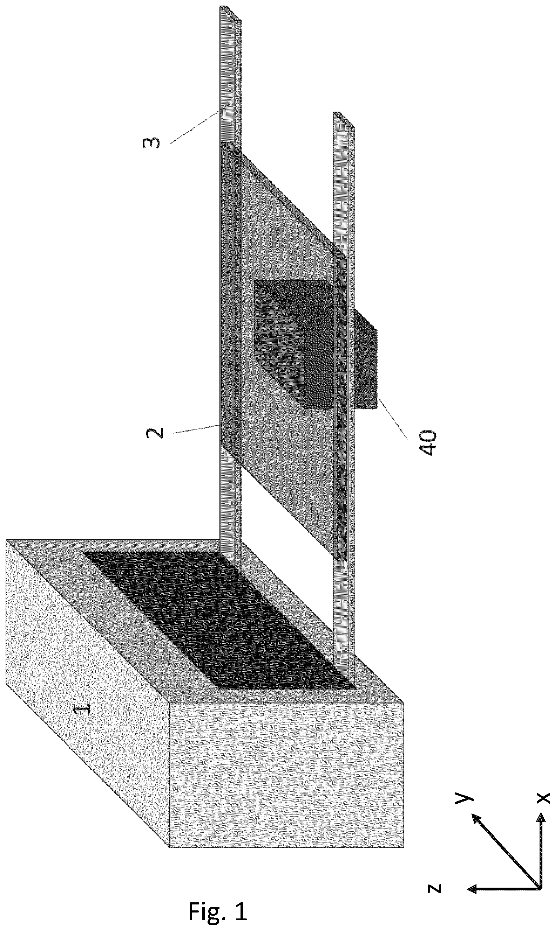

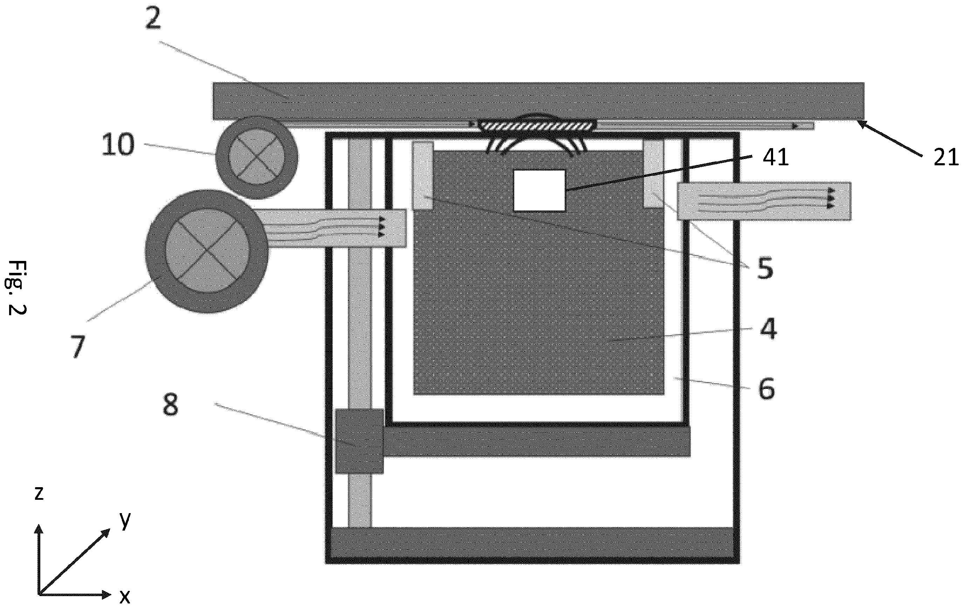

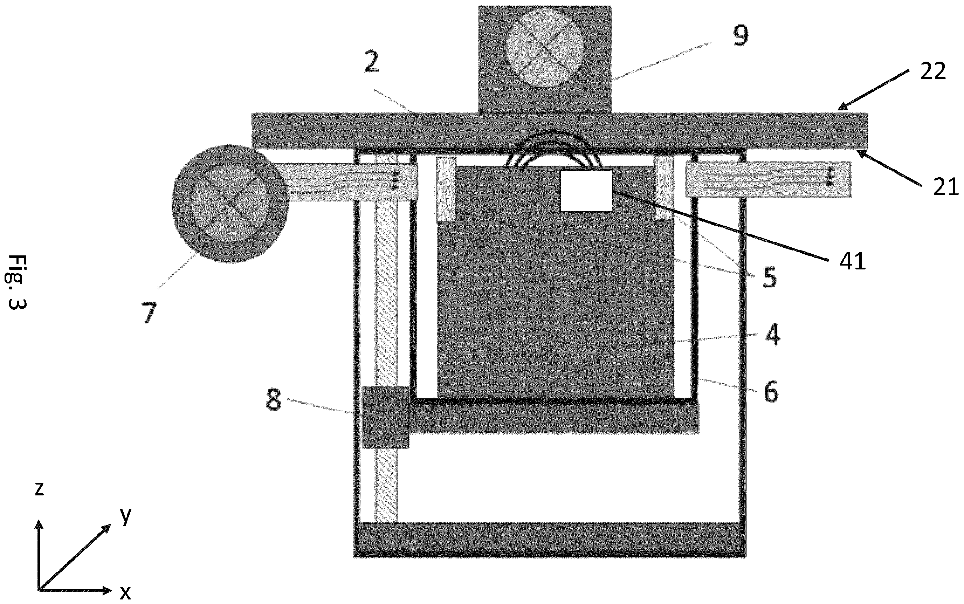

Figure 1 shows the position of the NMR measuring station in the lamination line, where 1 is a laminator, 2 is a polymer sample, 3 is a supporting structure, 40 is a measuring station, which is further detailed in Figures 2 and 3.

Figure 2 shows the first schematic embodiment of a device according to the second aspect of the present invention for the inline and offline mode of measurements. The inline implementation can take advantage of the hot products (polymer samples) 2 coming out of the laminator 1, resting on the supporting structure 3, to boost the sensitivity of the NMR cross-linking density determined by the measuring station, consisting of an NMR sensor 40, with a magnet 4, radiofrequency coil 41, spectrometer, PC, control and analysis software (not shown) for determination of the microstructural parameter. However, the changing sample-surface temperature occurring upon cooling must be taken into account by the precise temperature measurement at the moment of the measurement, using temperature sensors 5. Also, the magnet temperature can be controlled by the temperature sensors 5. To decouple the NMR magnet 4 from the environment thermally and magnetically, the magnet 4 is shielded by a shielding box 6 with a thickness lower than the maximal penetration depth of the measuring system. With time, the magnet 4 can still be heated up due to the heat transfer through the shielding box 6. To avoid this, the magnet 4 is cooled down by the active cooling device 7, which is to keep the magnet temperature constant. To vary the depth of the measuring position, the NMR sensor can be attached to the positioning device 8 to be moved along the z direction. The measurements will be performed on the moving samples, which renders the observed NMR cross-linking densities to be spatial averages over the sample area, depending on the production rate and the size of the used rf coil. The sample and magnet temperatures must be continuously monitored and recorded.

Also, the polymer sample 2 can be tempered from the sample bottom using a hot air blower 10 (heating device) for the local heating of the lower sample surface 21.

Figure 3 shows a second schematic embodiment of a device according to the second aspect of the present invention for both an inline as well as an offline mode of measurements. In contrast to the embodiment of Figure 2, the device does not have the heating device 10 in form of an air blower, but rather has a halogen lamp 9 (heating device) arranged at the second upper surface 22 of the polymer sample 2 (pointing away from the NMR sensor), wherein the heating device 9 is configured for tempering the polymer sample 2 prior to the measurement. Also, the heating device 9 might comprise a second thermometer for measuring the temperature at the upper surface 22 of the polymer sample 2 and to control the heating source 9 during the tempering of the polymer sample 2.

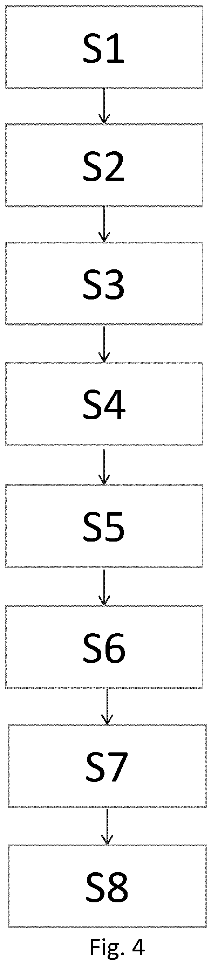

Figure 4 shows schematically a diagram of a method according to an embodiment of the first aspect of the present invention.

In a first step S1, the polymer sample 2 is tempered by means of the heating device 10 or of the heating source 9, in order to uniformly raise the temperature of the polymer sample to a predetermined temperature before starting the measurement. If the polymer sample 2 is directly coming from the lamination device, however, the tempering step can be skipped, as the polymer sample 2 is already heated up.

In a second step S2, the temperature of the surface 21 and/or of the second surface 22 of the polymer sample 2 is measured by the temperature sensor 5 or/and by the second thermometer being integrated within the heating source 9.

In a third step S3, a magnetic field is applied to the polymer sample 2 by means of the magnet 4. The magnetic field can either be constant or be a stray magnetic field with a one, two or three dimensional gradient, in order to excite a volume of a desired shape containing information on the microstructure of the polymer sample 2.

In a fourth step S4, a radio-frequency pulse sequence is applied to the polymer sample 2 by means of the radio-frequency coil 41 in the presence of the external magnetic field with a defined gradient, provided by the magnet 4.

In a fifth step S5, a signal response from the polymer sample 2 is detected by the radio-frequency coil 41.

In a sixth step S6, the signal response detected by the radio-frequency coil 41 is elaborated by the computing device of the device in order to extract a characteristic microstructure-dependent parameter of the nuclear magnetic resonance spectroscopy applied to the polymer sample, such as the spin-lattice relaxation time ($T_1$), the spin-spin relaxation time ($T_2$, to be more exact, $T_{2eff}$ in the inhomogeneous external and polarization magnetic fields), the proton density of the polymer sample, or/and the dipole-dipole couplings. From one or more of the above cited parameters, the microstructure of the polymer sample can be determined.

In a preferred embodiment, a pulse sequence, sensitive to a $T_{2eff}$ parameter is applied (e.g. based on the CPMG experiment (Carr, Purcell, Meiboom, Gill)). The $T_{2eff}$ parameter is either directly extracted from the signal response, for example, by performing a signal fitting of the signal response, wherein the signal fitting is in particular a sum of exponentials, in particular of stretched exponentials. Other operations that can be performed on the signal response or on the information extracted from the signal response by means of the computing device are a normalisation of the signal response, followed by the integration over time, or an echo-summation of the signal response (normalized or non-normalized).

After the elaboration and the performing of the operations on the signal response in step S6, a microstructure-dependent parameter of the polymer sample 2 is extracted from the elaborated signal response (step S7).

The microstructure-dependent parameter is typical for the polymer sample 2 and can relate, for example, to the cross-linking degree, crystallinity and/or to the water content within the polymer sample 2.

In a further step S8, the microstructure-dependent parameter is compared with a reference information obtained during a calibration run of the microstructure-dependent parameter in question. For example, for determining the cross-linking degree, a polymer sample having a certain composition can be examined, for different cross-linking times, by means of a Soxhlet extraction and, after such analysis, the same polymer sample having the same composition and with the same cross-linking times can be analysed by means of the method described with the diagram of Figure 4. After the analysis with the nuclear magnetic resonance spectroscopy, there is then a calibration chart for determining the cross-linking degree of any polymer sample having the same composition as the one for which the calibration procedure has been performed. Thus, it is possible to non-destructively analyse a large number of polymer samples all having the same composition in a time and resource efficient manner.

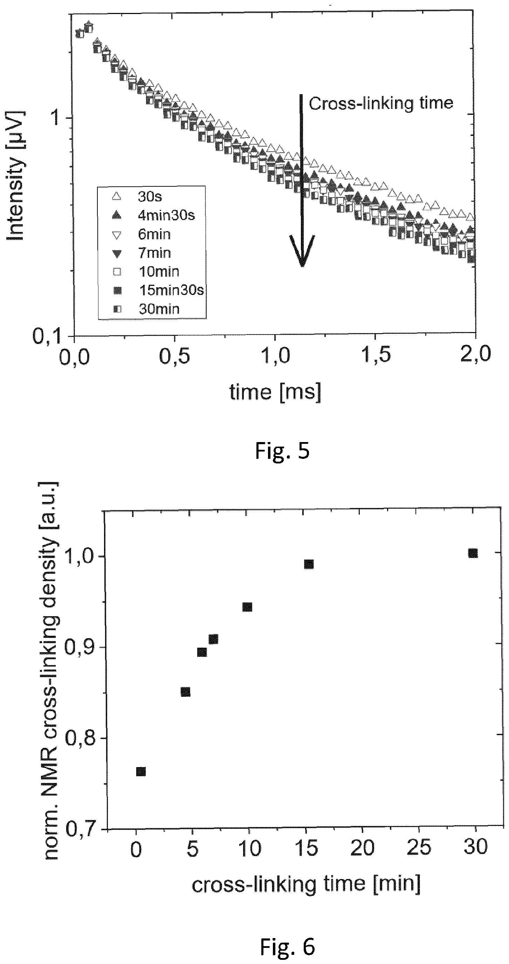

Figure 5 shows a diagram of a signal response intensity over time for a polymer sample for different cross-linking times. The polymer sample 2 for the measurements, whose results are depicted in Figure 5 is an ethylene vinyl acetate sample laminated using a number of different cross-linking times, namely 30 seconds, 4 minutes and 30 seconds, 6 minutes, 7 minutes, 10 minutes, 15 minutes and 30 seconds and 30 minutes. On the x-axis there is depicted the time at which the signal response is measured in ms. The y-axis represents the signal intensity of the signal response as measured by the radio-frequency coil 41 in $\mu V$. As it can be seen from Figure 5, the signal response has a steeper decay for longer cross-linking times. The measurement time was 6 minutes, but it was found that it can be reduced down to 1.2 s without sacrificing the measurement precision. The standard deviation for the results shown in Figure 5 is found to be at around 1%. Reducing the measuring time to the millisecond scale is possible, although the repeatability and the sensitivity of the results would decrease from the reduced measuring time.

Figure 6 shows a diagram of an NMR cross-linking degree for the polymer samples of Figure 5, obtained as a reciprocal of an echo summation ($T_{2eff}$-weighted amplitude) within the whole acquired time range, normalized to the maximal value at the lamination time of 30 min. A time range for the echo summation can be freely chosen to achieve maximal signal sensitivity towards microstructure.

Figure 6 shows on the x-axis the cross-lining time is minutes and on the y-axis the normalised NMR cross-linking degree as determined according to the procedure explained above.

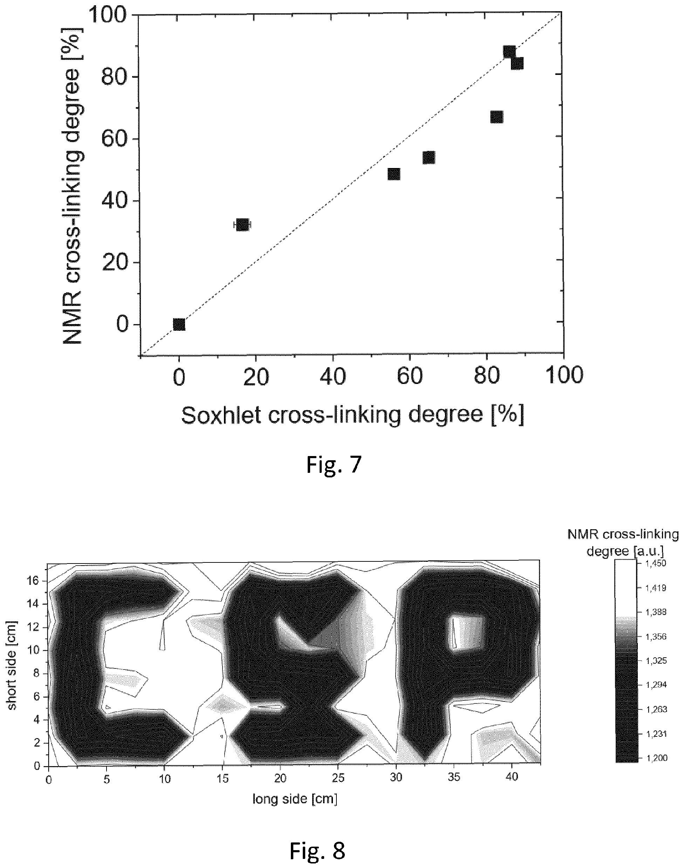

Figure 7 shows a comparison of the NMR cross-linking degree with a Soxhlet cross-linking degree for the same polymer samples as the ones used for the measurements of Figure 5 and Figure 6. The Soxhlet cross-linking degree is determined by means of a Soxhlet extraction process on the polymer samples. As it can be seen from Figure 7 there is a good correlation between the cross-linking degree as obtained from the Soxhlet extraction and the cross-linking degree obtained from the nuclear magnetic resonance spectroscopy with a method according to the first aspect of the present invention, even if the measuring time is reduced to 1,2 seconds. Therefore, the method according to the first aspect of the present invention delivers good results in a time and resource efficient manner, without the need for performing destructive analyses on the polymer sample.

Figure 8 shows a nuclear magnetic resonance spectroscopy image of an ethylene vinyl acetate film as a polymer sample with dimensions 42,5 centimetres by 17 centimetres, in which lamination defects in form of the letters CSP were purposely introduced during the lamination process. For the image of Figure 8, an inhomogeneous magnetic field having a defined linear gradient component in the z-direction is used. The mapping is performed by moving the sample. The measuring time is set at 1,2 seconds per position and a total of 145 positions are measured, thus leading to a total measuring time of 2 minutes and 54 seconds. As it can be seen from Figure 8, the method according to the first aspect of the present invention is able to correctly identify the lamination defects introduced in the body of the polymer sample.

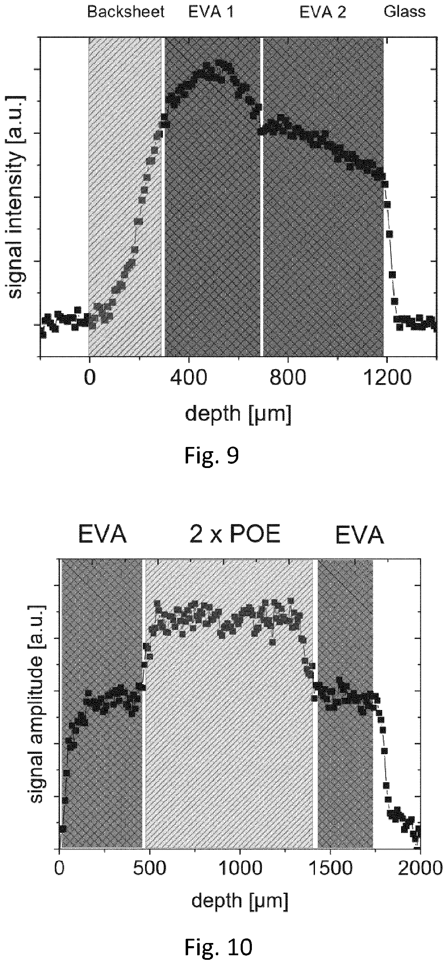

Figure 9 shows a normalised signal response intensity, obtained by an echo summation, inversely related to a cross-linking degree within a polymer sample having multiple layers, namely a glass layer, a first ethylene vinyl acetate layer, a second ethylene vinyl acetate layer and a backsheet being laminated together for five minutes, which corresponds to a Soxhlet cross-linking degree of circa 50%. Such multilayer structure might be typical for a photovoltaic module, wherein in a photovoltaic module a photovoltaic cell is provided between the first and the second ethylene vinyl acetate layers.

For the measurement represented in Figure 9, an inhomogeneous magnetic field having a defined linear gradient component in the z-direction is used. As can be seen from the diagram of Figure 9, the cross-linking degree decreases from the glass layer towards the backsheet, due to the one sided heating at the glass layer, which leads to a temperature gradient between the glass layer and the backsheet. This temperature gradient results then in a cross-linking degree gradient across the multilayer polymer sample.

The method according to the first aspect of the present invention is also proven to provide reliable results for other materials other than the ones used for the multilayered polymer sample of Figure 9. For example, in Figure 10, a multilayer polymer sample having a first ethylene vinyl acetate layer, a second ethylene vinyl acetate layer and a polyolefin elastomer layer sandwiched between the two ethylene vinyl acetate layers is exposed to the method according to the first aspect of the present invention and the cross-linking degree is measured. On the x-axis the depth at which the cross-linking degree is measured is shown and on the y-axis the normalised signal intensity of the signal response, being inversely proportional to the cross-linking degree, is reported. The difference in the normalised signal intensity is however not only due to the different cross-linking degree, but also due to the different spin-spin relaxation times being characteristic of each material.

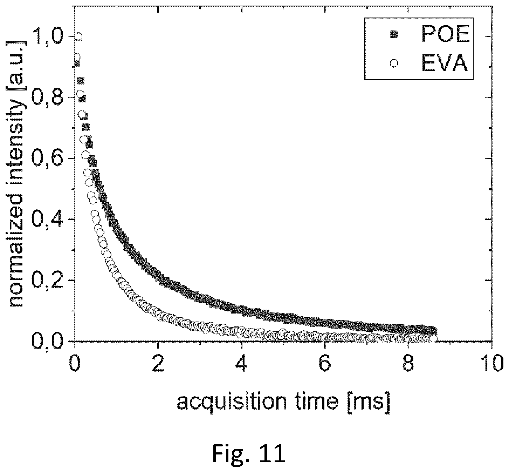

In Figure 11, a diagram showing the normalized intensity of the signal response from a polymer sample being made of polyolefin elastomer and from a polymer sample being made of ethylene-vinyl acetate copolymer is shown in relation to the acquisition time. As it can be seen from Figure 11, the two polymer samples have a different response, which is correlated with the different spin-spin relaxation times, intrinsically defined by the intrasegmental arrangement of protons as well as segmental mobilities.

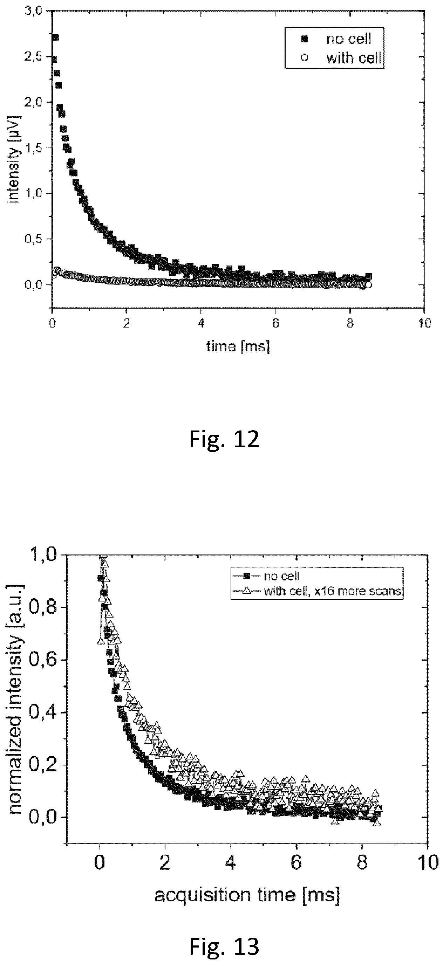

Figure 12 shows a diagram with the intensity of a signal response from an EVA film with or without a photovoltaic cell, in order to understand the effect of paramagnetic materials on the measurement method according to the first aspect of the present invention. In the polymer samples (EVA encapsulant) used for the measurement of Figure 12, the photovoltaic cell contains aluminium, a paramagnetic material. As it can be seen in Figure 12, the signal decay is much stronger in the presence of paramagnetic materials, such as aluminium, wherefore an overall signal intensity reduction is observed. The signal intensity reduction in Figure 12 is of a factor 16.

In Figure 13, the normalized intensity of the signal response against the acquisition time is shown for a measurement performed on the same polymer sample as in Figure 12. In the example of Figure 13, however, for the EVA film with the photovoltaic cell the scans are increased by a factor 16. As it can be seen from Figure 13, the decay of the normalised signal intensity from the signal response of the EVA film with the photovoltaic cell became effectively slower as compared to the EVA film measured without the photovoltaic cell.

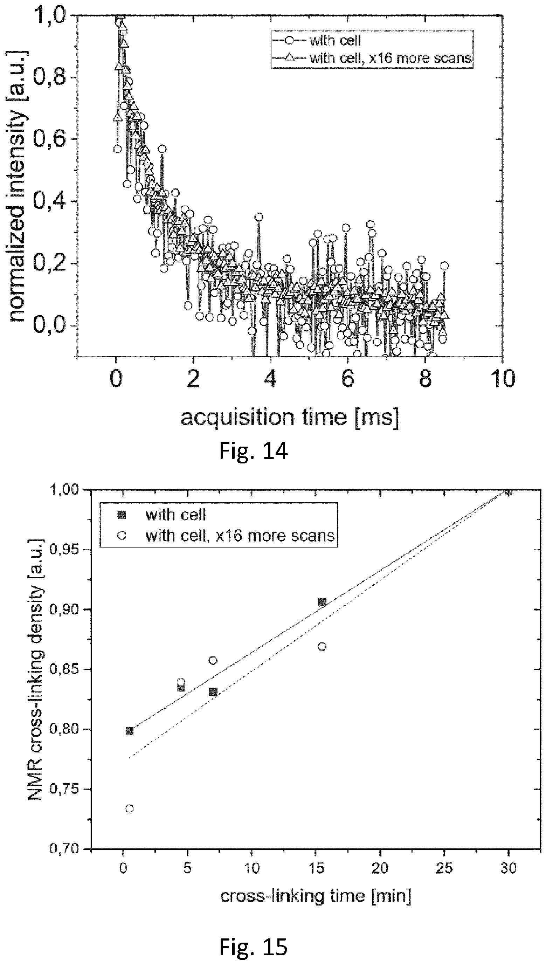

Also, the noise to signal ratio is reduced when incrementing the scans by a factor 16, as shown in Figure 14, where the normalised intensity of the signal response of a polymer sample being an EVA film with a photovoltaic cell is compared for the case in which the number of scans is increased by a factor 16 and for the case in which the number of scans is not increased by a factor 16.

Figure 15 shows a comparison of the NMR cross-linking degree against the cross linking time for EVA films measured in the proximity of a photovoltaic cell, laminated using different cross-linking times, for the case in which the number of scans is increased by a factor 16 and for the case in which the number of scans is not increased by a factor 16. Surprisingly, the inventors found out that the number of scans only marginally affect the sensitivity of the method according to the first aspect of the present invention with regard to the cross-linking degree even in the presence of a photovoltaic cell containing paramagnetic material. The linear slope for modelling the NMR cross-linking degree against the cross-linking time decreases by only 10% when 64 scans are performed instead of 1024, thus reducing the measuring time from 6 minutes and 40 seconds to only 25 seconds in the example of Figure 15. Thus, the lamination control of photovoltaic modules in the region of photovoltaic cells can be performed in a time and resource efficient manner.

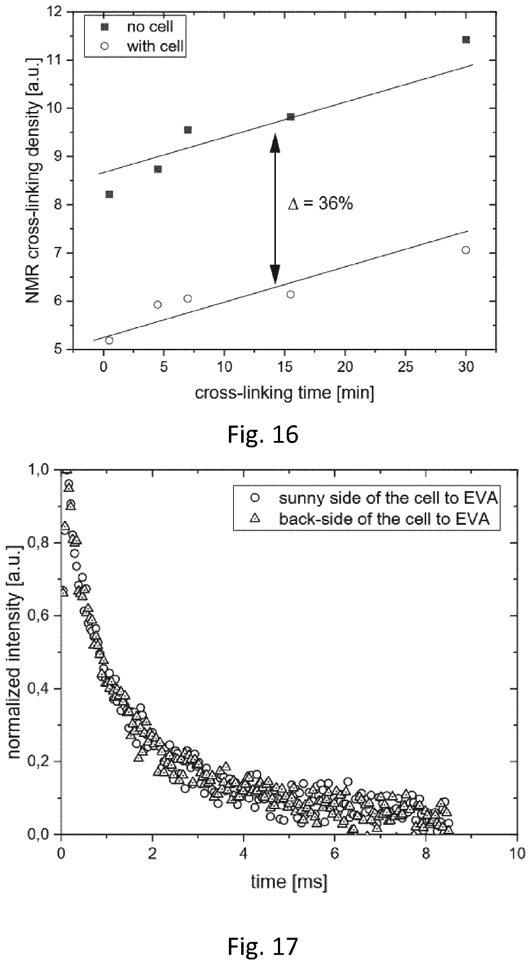

The values for the NMR cross-linking degree are compared for EVA films measured with or without a photovoltaic cell in Figure 16 for different cross-linking times. As it can be seen in Figure 16, both data sets have approximately the same relative difference of around 36% for all cross-linking times, caused by the use of paramagnetic material, in this case aluminium, in the photovoltaic cell. Therefore, two-dimensional mappings of the cross-linking distribution can be realised over the whole module area, both in regions where the photovoltaic cell is present as well as in regions where the photovoltaic cell is not present by applying the known difference as a correction factor for either of the regions.

In Figure 17 the effect of the sunny side of a photovoltaic cell and the backside of the photovoltaic cell on the normalised intensity of the signal response is shown. As it can be seen, no appreciable effect can be found, so that orientation of the cell during the measurement is not relevant.

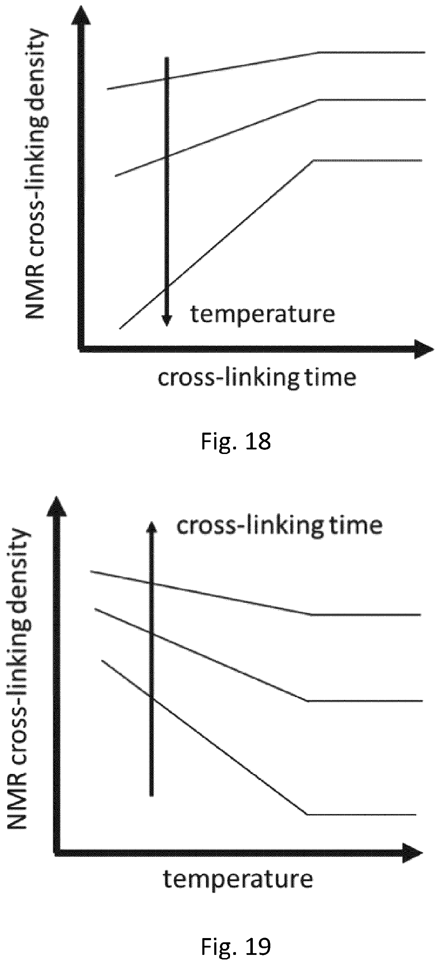

Figures 18 and 19 are a schematic representation of the effect of the sample temperature on the NMR cross-linking degree as derived from the signal response. The influence of the sample temperature on the NMR cross-linking degree as derived from the signal response is due to the fact that the NMR signal (signal response) is temperature dependent because of the changes in the molecular mobility, which cause the averaging out of nuclear magnetic resonance specific interactions, such as residual dipolar couplings, that drive the signal decay. Therefore, the corresponding NMR cross-linking degree as derived from the signal response is an apparent quantity that requires the knowledge of the sample temperature to be correctly interpreted.

Figure 18 represents schematically the NMR cross-linking degree as derived from the signal response against the cross-linking time for different temperatures. As the temperature increases, polymer segments become less dynamically constrained, especially due to the melting of the crystalline structures. Therefore, the sensitivity of the method according to the first aspect of the present invention increases with increasing sample temperature, whereby a plateau of the sensitivity is expected to be reached above 100 °C, depending on the polymer sample.

Figure 19 represents schematically the NMR cross-linking degree as derived from the signal response against the sample temperature for different cross-linking times.

**Claims**

**1.** Method for analysing a polymer sample (2), the method comprising:

  providing a polymer sample (2);
  tempering a polymer sample (2);
  measuring a surface temperature of the polymer sample (2) ;
  subjecting the polymer sample (2) to a magnetic field of a magnet (4);
  applying a radiofrequency pulse sequence to the polymer sample (2);
  obtaining a radiofrequency signal response from the polymer sample (2);
  determining a microstructure-dependent parameter of the polymer sample (2) on the basis of the signal response.

2. Method according to claim 1, wherein the polymer sample (2) is a multilayer sample with at least two layers, wherein the two layers are in particular an ethylene vinyl acetate layer and a polyolefin elastomer layer.

3. Method according to claim 2, wherein the polymer sample (2) is photovoltaic module, in particular a glass-backsheet module or a glass-glass module.

4. Method according to any of the preceding claims further comprising:

  applying a CPMG pulse sequence;
  performing a saturation recovery or an inversion recovery on the signal response; and
  extracting a spin-lattice relaxation time.

5. Method according to any of the preceding claims further comprising:
  extracting a proton density and/or a spin-spin relaxation time from the signal response.

6. Method according to any of the preceding claims further comprising:
  extracting dipole-dipole couplings from the signal response.

7. Method according to any of the preceding claims further comprising
  performing a signal fitting of the signal response, wherein the signal fitting is in particular based on a sum of exponentials, in particular of stretched exponentials.

8. Method according to claim 5 further comprising normalizing the signal response based on a microstructure-dependent parameter, to a maximum intensity at an initial time and integrating the signal response over time to obtain an amplitude-weighted $T_{2eff}$ average.

9. Method according to any of the preceding claims further comprising performing an echo-summation of the signal response.

10. Method according to claim 9 further comprising inverting the $T_{2eff}$ value.

11. Method according to any of the preceding claims further comprising comparing the microstructure-dependent parameter with reference information.

12. Method according to any of the preceding claims, wherein the magnetic field comprises a gradient.

13. Method according to claim 12, wherein the gradient is a one-dimensional, a two-dimensional or a three-dimensional gradient.

14. Device for carrying out a method according to any of the previous claims, the device comprising:

  a supporting structure (3) for supporting the polymer sample (2);
  a temperature sensor (5) for measuring a surface temperature of the polymer sample (2);
  a magnet (4) generating a magnetic field at a location of the polymer sample;
  a radio-frequency coil (41) for sending radio-frequency pulse sequences at the location of the polymer sample (2) and for receiving a radio-frequency signal response of the polymer sample (2); and
  a computing device for determining a microstructure-dependent parameter of the polymer sample (2) on the basis of the signal response.

15. Device according to claim 14 further comprising at least one of

  a heating device (9, 10) for heating the polymer sample (2), wherein it is preferably provided that the heating device (9, 10) is a halogen lamp or a hot-air ventilator;
  a further temperature sensor (5) for measuring a temperature of the magnet (4);
  a cooling device (7) for cooling the magnet (4);
  a thermally shielding box (6) for thermally decoupling the magnet (4) from the surrounding environment;
  a positioning device (8) for moving the magnet (4) along a direction (z) being perpendicular to a surface (21, 22) of the polymer sample (2); wherein the supporting structure (3) configured for allowing a relative movement between the polymer sample (2) and the magnet (4).

Fig. 1

Fig. 2

Fig. 3

S1

↓

S2

↓

S3

↓

S4

↓

S5

↓

S6

↓

S7

↓

S8

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Europäisches
Patentamt
European
Patent Office
Office européen
des brevets

**EUROPEAN SEARCH REPORT**

Application Number

EP 23 21 9911

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | S. ANFEROVA: "Thermo-oxidative aging of elastomers: a temperature control unit for operation with the NMR-MOUSE", APPLIED MAGNETIC RESONANCE., vol. 27, no. 3-4, 1 September 2004 (2004-09-01), pages 361-370, XP093160988, AU ISSN: 0937-9347, DOI: 10.1007/BF03166737 Retrieved from the Internet: URL:https://link.springer.com/content/pdf/10.1007/BF03166737.pdf> | 1,5, 11-14 | INV. G01N24/08 G01R33/31 G01R33/44 G01N33/00 G01N33/44 H01L31/048 |
| Y | * pages 361-367 and 370 * | 2,4,6-10 | ADD. G01R33/30 G01R33/46 |
| A |  | 3 | |
| Y | BLUMICH B ET AL: "Mobile single-sided NMR", PROGRESS IN NUCLEAR MAGNETIC RESONANCE SPECTROSCOPY, PERGAMON PRESS, OXFORD, GB, vol. 52, no. 4, 1 May 2008 (2008-05-01), pages 197-269, XP022589395, ISSN: 0079-6565, DOI: 10.1016/J.PNMRS.2007.10.002 [retrieved on 2007-12-28] * Sections 3.1.4 and 3.2 Text on p.214 and on p.241; figures 26,47,49 * | 2,4,6-10 | |

**TECHNICAL FIELDS SEARCHED (IPC)**

G01N
G01R

The present search report has been drawn up for all claims

| Place of search | Date of completion of the search | Examiner |
|---|---|---|
| Munich | 14 May 2024 | Lebar, Andrija |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

**Application Number**

EP 23 21 9911

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-14

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION**
**SHEET B**

Application Number

EP 23 21 9911

The Search Division considers that the present European patent application does not comply with the requirements of unity of invention and relates to several inventions or groups of inventions, namely:

```
1. claims: 1-14

    A method for analysing a photovoltaic module polymer sample

1.1. claims: 1-3

    A method for analysing a photovoltaic module polymer sample

1.2. claims: 4-14

    Additional features that are trivial, unimportant to the
    invention or generally known in the technical field of the
    invention
                    - - -

2. claim: 15

    A device for analysing a polymer sample with magnetic
    resonance with means to keep the magnet temperature constant
                    - - -

Please note that all inventions mentioned under item 1, although not
necessarily linked by a common inventive concept, could be searched
without effort justifying an additional fee.
```